# EUROPEAN PATENT SPECIFICATION

(11) **EP 1 372 755 B1**
(45) Date of publication and mention of the grant of the patent: **20.08.2008**
(21) Application number: 02706688.5
(22) Date of filing: 15.03.2002
(51) Int. Cl.: A61M 1/00

(54) **AN APPARATUS FOR FLUSHING PERIPHERAL ORGANS IN HUMANS OR ANIMALS**
VORRICHTUNG ZUR SPÜLUNG DER PERIPHEREN ORGANE IN MENSCHEN ODER IN TIEREN
APPAREIL DESTINE A RINCER DES ORGANES PERIPHERIQUES CHEZ L'HOMME OU CHEZ L'ANIMAL

(30) Priority: 21.03.2001 DK 200100465; 28.10.2001 DK 200101590; 07.12.2001 DK 200101819
(43) Date of publication of application: 02.01.2004
(73) Proprietor: Curatio ApS, 800 Arhus C (DK)
(72) Inventor: KIRKEGAARD, Jesper, DK-7400 Herning (DK); NIELSEN, Rasmus, Lundsgaard, DK-8000 Arhus C (DK); KIDMOSE, Casper, DK-8000 Arhus C (DK); ANDERSEN, Henrik, DK-8230 Abyhoj (DK); FONS, Peter, c/o Helene Fons, DK-6070 Christiansfeld (DK); HARDING, Hans, DK-9210 Alborg So (DK)
(74) Representative: Nielsen, Leif
(86) International application number: PCT/DK2002/000174
(87) International publication number: WO 2002/074363

(56) References cited:
- EP-A- 0 093 405
- EP-A- 0 197 273
- WO-A-99/11192
- DE-A- 3 207 058
- DE-U- 29 606 600
- LU-A- 82 873
- US-A- 5 071 104
- US-A- 5 941 859
- US-A- 6 125 843

## Description

The invention relates to an apparatus for flushing peripheral organs in humans and animals, wherein liquid is conveyed from a reservoir under pressure to a discharge branch via a hose or the like to the peripheral organs.

Such an apparatus is e.g. particularly suitable for cleaning contaminated wounds caused e.g. by fall accidents.

The technique used in the past for the flushing of peripheral organs, such as cleaning of wounds in case of skin lesions, is typically based on the use of tap water which is sprayed on the wound directly from a hose coupled to the drinking water supply.

It is also common to use a syringe made for the purpose, into which the tap water or sterile salt water has been sucked beforehand, e.g. from a bowl which is positioned near the patient.

It is common in this connection that the liquid jet used for the flushing is ejected in a smooth, non-pulsating stream.

Now, an object of the invention is to provide a method by which flushing of an organ may be carried out more effectively.

The object of the invention is achieved by the apparatus of claim 1.

With a view to optimizing flushing of an organ additionally, it is an advantage if, as stated in claim 2, the discharge nozzle comprises a hand-held unit from which the volume and the jet shape of the liquid may be adjusted, said hand-held unit having a roll closure device, and the discharge nozzle has one or more holes. The quantity of the pulsating flow and its jet pattern may hereby be adapted to a given task.

For easy replacement or exchange of a liquid which is to be discharged from the apparatus according to the invention, it is advantageous if, as stated in claim 3, the reservoir is formed by a liquid-tight bag, which is preferably made of a plastics material, such as PVC or polyurethane. Such bags are usually made as disposable bags with different contents.

To ensure that the liquid supplied from the discharge nozzle, after having been in contact with e.g. a wound, does not contaminate the surroundings, it is expedient if, as stated in claim 4, the hand-held unit is terminated with an adjustable shield which is made of a preferably transparent material, such as PVC or polyurethane.

With a view to controlling the pulsation and the flow of the liquid through the apparatus as best as possible, it is an advantage if, as stated in claim 5, the hose connecting the reservoir with the hand-held unit is made of a flexible and preferably transparent material, such as silicone rubber, polyurethane or PVC.

When flushing e.g. wounds where flushing is to be carried out over an extended period of time, it is an advantage if, as stated in claim 7, the apparatus is provided with an assembly which is capable of heating the liquid in the liquid reservoir to a predetermined temperature and keeping the temperature of the liquid within a given tolerance range, and the temperature being kept is preferably between 20 and 40 degrees Celsius, more preferably 37 degrees Celsius.

When, as stated in claim 14, a shield is arranged around the discharge branch, it is ensured that liquid ejected on an organ is not spread, thereby obviating the risk of spreading of impurities in the form of e.g. bacteria.

Additional expedient embodiments of the invention are defined in the other dependent claims.

The invention will now be explained more fully with reference to the drawing, in which
- fig. 1: shows a basic sketch of a setup of an apparatus according to the invention in a first embodiment,
- fig. 2: shows a pulsator for use in the setup of fig. 1 in a first embodiment,
- fig. 3: shows a pulsator for use in the setup of fig. 1 in a second embodiment,
- fig. 4: shows a pulsator for use in the setup of fig, 1 in a third embodiment,
- fig. 5: shows a setup of an apparatus according to the invention in a second embodiment,
- fig. 6: shows a setup like in fig. 5, but with a roll pump in a second embodiment, while
- fig. 7: shows a hand-held unit for use in the setups of figs. 1, 5 and 6.

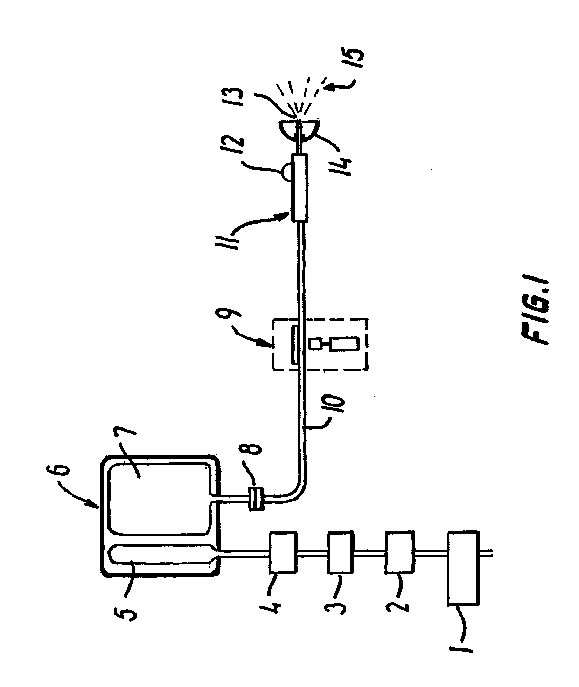

In fig. 1, the numeral 1 designates an air pump which is connected via a hose with a pressostat 2, a safety valve 3, a pressure relief valve 4 and with a pressure bag 5.

The pressure bag 5 is arranged in a chamber 6, which also accommodates a reservoir in the form of a bag 7 with liquid, which may be sterile.

The pressure bag 5 may be caused to change its volume by filling of air or e.g. liquids, including water.

A hose 10 extends from the bag 7 and is connected via a connector 8 and a pulsator 9 with a hand-held unit 11, through which the hose 10 extends. The hand-held unit has a roll 12 which, as will be explained later, can adjust the amount of liquid which is discharged from the hose 10 from the hand-held unit 11 to a discharge nozzle 13. As will also be seen, the hose is surrounded by a protective shield 14 in the vicinity of the nozzle 13.

The setup in fig. 1 operates in the following manner:

Pressure application from the air pump 1 expands the volume of the pressure bag 5 in the chamber 6, which thereby exerts a pressure against the bag 7 that contains the liquid to be discharged through the nozzle 13.

Activation of the pulsator 9 causes pulsation of the liquid so as to provide a pulsated flow out through the nozzle.

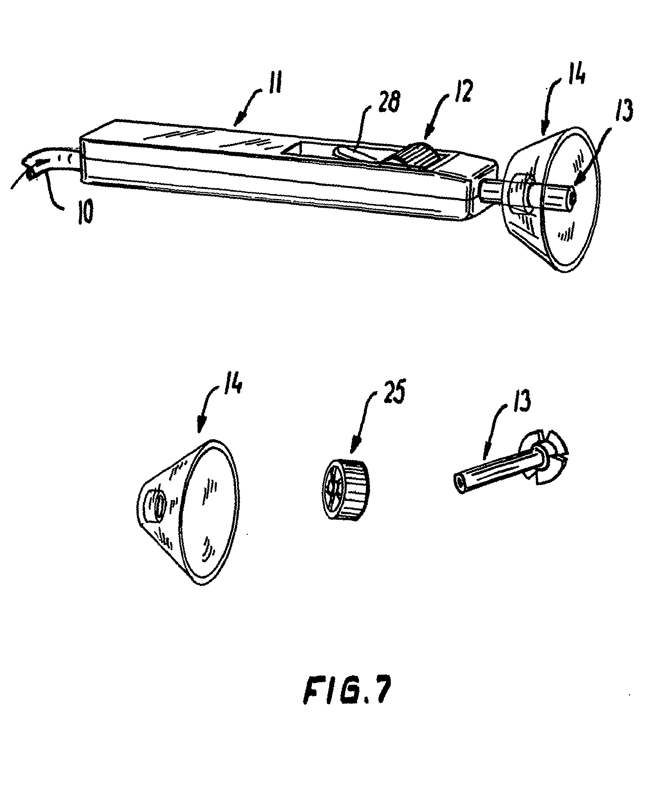

The amount of the liquid which is to be discharged from the nozzle 13 may be adjusted by means of the hand-held unit, as the wheel on the hand-held unit is adapted to squeeze the hose 10 by displacement, cf. the explanation in connection with fig. 7.

The pressostat 2 is used for adjusting the pressure in the pressure bag 5, while the safety valve 3 is adapted to open if the pressure exceeds a max. pressure, which may be determined by the maximum permissible pressures of the components incorporated in the setup.

The pressure relief valve 4 is adapted to relieve the pressure from the pressure bag 5, which is desirable if e.g. the bag 7 is to be replaced.

The apparatus is used in practice in that the nozzle with a shield 14 is directed toward a human's or animal's organ which is to be flushed. When the liquid is discharged in a pulsating manner against the organ, the protective shield ensures that the liquid reflected from the organ is not ejected in all directions with the consequent risk of spreading of impurities.

Below, various embodiments of the functions of the components incorporated in the setup of fig. 1 will be explained.

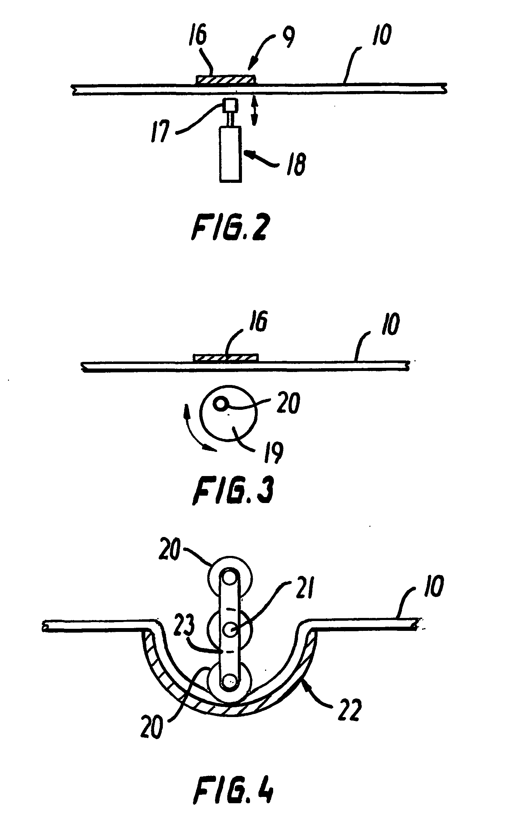

Fig. 2 shows an embodiment of the pulsator 9 in fig. 1. It consists of a mechanical stop 16 which is positioned close to the hose 10. A piston 17 may be moved by means of an electromagnet 18, so that the hose is squeezed between the piston 17 and the mechanical stop 16. Suitable selection of the current fed to the electromagnet 18 allows a pulsation to be established in the hose 10 which is proportional to the frequency of the current fed to the electromagnet.

Fig. 3 shows another embodiment of the pulsator. This embodiment, too, includes the mechanical stop 16 which is positioned close to the hose 10. An eccentrically rotating disc or ball bearing 19 having a shaft 20 is driven by an electric motor, which will affect the hose with a frequency which is proportional to the speed of rotation of the wheel.

Fig. 4 shows an embodiment of the pulsator in which a roll pump is used. This roll pump has inter alia a semi-circular housing along whose inner side the hose 10 extends. As will also be seen, the roll pump 2 has rolls 20 which are mounted on a rod 23. The rod 23 with rolls 20 is driven by a motor around a shaft 21, which causes the rolls to alternately squeeze the hose 10 and thereby provide a pulsating effect. Even though the pump is just shown with two rolls, nothing prevents mounting of more rolls, preferably two or more, which will then be mounted on an additional rod (not shown) perpendicular to the shown rod.

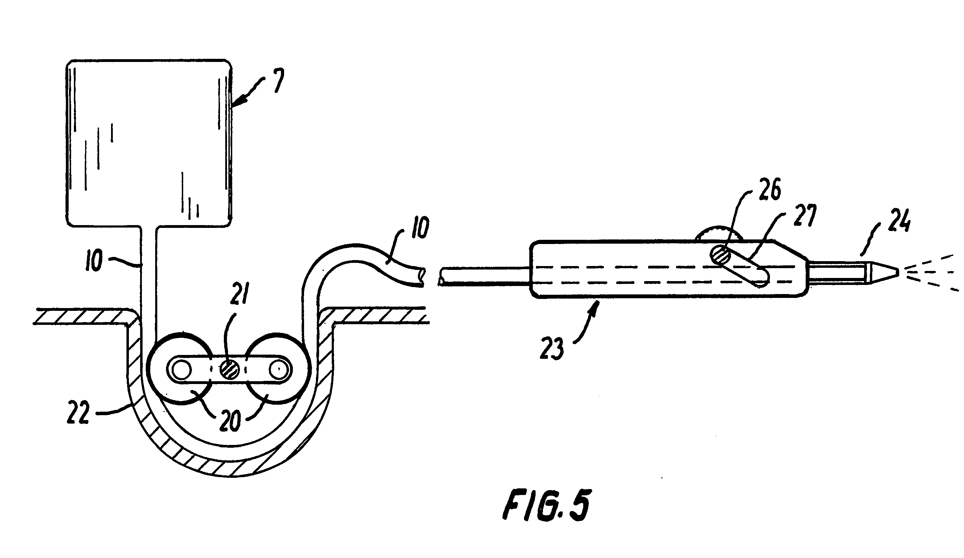

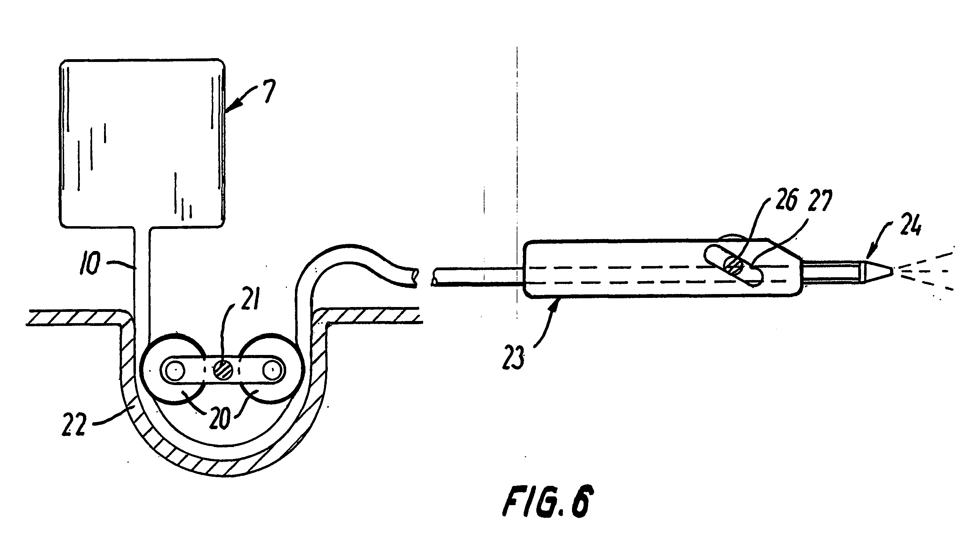

Figs. 5 and 6 show a basic setup of the apparatus in a second embodiment in which the pulsator of fig. 5 is incorporated. Both figures include the roll pump with rolls 20 mounted on a rod that can rotate about the shaft 21, as explained in connection with fig. 5, but now connected at its inlet side via a hose 10 connected with a liquid reservoir in the form of a bag 7 corresponding to the one shown in fig. 1. At the outlet side of the pump where the hose 10 extends, there is connected a hand-held, adjustable flow control 23 which is terminated by a nozzle 24 from which liquid may be dispensed. This flow control has a roll 26 which may be moved manually in a roll groove 27, whereby the flow of liquid may be adjusted in that the roll 27 squeezes the hose 10 completely or partly when the roll moves in the roll groove.

In fig. 5, the roll is not in contact with the hose 10, thereby providing maximum flow, while in fig. 6 it is shown in a position in which it squeezes the hose 10 partly and thereby provides a reduced flow.

Fig. 7 is a more detailed view of the hand-held unit 11 from fig. 1, which consists of the pulsator, e.g. as shown in figs. 5 and 6, as well as the connected nozzle 13 and the protective shield 14. As will be seen, the roll 12, the nozzle 13 and the protective shield 14 mounted on the nozzle are included. The roll 12 may be moved in the shown groove 18 and thereby limit the flow discharged from the nozzle 13, as explained in connection with figs. 5 and 6. The bottom of the figure shows the protective shield 14 and the nozzle 13 separated from each other as well as a shown connecting part 25 for assembling the parts. It should moreover be noted that the protective shield may be moved longitudinally of the pipe connected with the nozzle.

The materials and characteristics of the parts according to the invention may e.g. be as follows:

The bag 7, which constitutes the liquid reservoir, may typically be made of a soft, transparent material, such as e.g. silicone rubber, polyurethane or PVC and will preferably have a volume between 0.5 and 3.0 litres, more preferably 1.0 litre.

As regards dimensions, the hose 10, which connects the liquid reservoir and the hand-held, adjustable unit 11, will preferably have an internal bore diameter of between 3 and 10 mm, with 5 mm as the most optimum dimension. The hose 10 is typically made of a visually transparent and flexible material, such as silicone rubber, polyurethane or PVC.

The chamber 6 may be a bag which is made of a strong, preferably transparent material capable of resisting an inner pressure. It is also preferred that the chamber 6 is provided with a flexible separation between the pressure bag 5 and the liquid bag 7. Moreover, the chamber 6 may be provided with a closure mechanism, such as a zipper that allows opening of the chamber for exchange of the liquid bag 7.

The pressure bag 5 is preferably made of a flexible material, such as rubber, so that the volume of the pressure bag will increase as a function of the pressure in the pressure bag.

A suitable dynamic liquid pressure measured after the discharge nozzle is between 4 and 15 psi, with 10 psi being used most frequently.

When the hand-held flow control is fully opened, the apparatus in a preferred embodiment will be capable of supplying a liquid flow of between 0.3 and 0.6 litre per minute, with 0.45 litre per minute being frequently preferred.

The discharge nozzle, which is mounted on the hose where the liquid is ejected from the apparatus, is shown with a straight nozzle opening in the enclosed figures. However, also other discharge nozzles having more than one nozzle hole may be used, and the nozzle holes may be angled relative to each other.

The discharge nozzle may also be extended with an adapter which changes the ejection characteristic of the ejected liquid. Such an adapter may e.g. be provided with three nozzle holes, with the central one extending in the centre axis of the adapter in the longitudinal direction, while the two others extend at an angle relative to the centre axis of the nozzle, thereby achieving a discharge liquid jet which consists of three jets that give a radiation spread in a plane.

Of course, the adapter must be constructed such that its internal side coupled to the discharge nozzle has a configuration which corresponds to the outer configuration of the discharge nozzle. The discharge nozzle and the adapter may be held in the coupled state by ordinary friction caused by an expedient tolerance of the dimensions of the individual coupled surfaces and an expedient configuration of these. The nozzle and the adapter may also be coupled together via threads or a snap locking system.

Thus, adapters may be manufactured for the discharge nozzle which have an arbitrary number of jet paths which may be angled arbitrarily relative to the centre axis of the nozzle system which extends in the longitudinal direction.

Finally, the apparatus may be powered by a battery, which may be rechargeable, or be fed with energy by connection to the lighting mains.

## Claims

1. An apparatus for flushing peripheral organs in humans and animals, wherein liquid is conveyed from a reservoir (7) under pressure to a discharge branch (13) via a hose (10) or the like to the peripheral organs, wherein a pulsator (9) is arranged between the reservoir (7) and the discharge branch (13), said pulsator being adapted to pulsate the liquid discharged from the discharge branch (13), **characterized in that** the apparatus comprises a pressostat for controlling the pressure and that the pulsator and the pressostat are individually adjustable for mutually independent regulation of the pressure and the pulsation.

2. An apparatus according to claim 1, **characterized in that** the discharge nozzle comprises a hand-held unit (11) from which the volume and the jet shape of the liquid may be adjusted, said hand-held unit (11) having a roll closure device (12), and that the discharge nozzle (13) has one or more holes.

3. An apparatus according to claims 1 - 2, **characterized in that** the reservoir (7) is formed by a liquid-tight bag which is preferably made of a plastics material, such as PVC or polyurethane.

4. An apparatus according to claims 1-3, **characterized in that** the hand-held unit (11) is terminated with an adjustable shield (14) which is made of a preferably transparent material, such as PVC or polyurethane.

5. An apparatus according to claims 1-4, **characterized in that** the hose (10), which connects the reservoir (7) with the hand-held unit (11), is made of a flexible and preferably transparent material, such as silicone rubber, polyurethane or PVC.

6. An apparatus according to claims 1-5, **characterized in that** the pulsator (9) is adapted to affect the hose (10) by a pulsating complete or partial squeezing.

7. An apparatus according to claims 1 - 6, **characterized in that** it is provided with an assembly which is capable of heating the liquid in the liquid reservoir to a predetermined temperature and keeping the temperature of the liquid within a given tolerance range, and that the temperature being kept is preferably between 20 and 40 degrees Celsius, more preferably 37 degrees Celsius.

8. An apparatus according to claim 6 or 7, **characterized in that** the squeezing of the hose (10) is provided by means of a closure mechanism (16, 17, 18), which is moved by the impact from an electromagnet (18) or by an electrically or hydraulically or pneumatically controlled motor or by the use of a preferably controllable roll pump (20, 21, 22) having one or more rolls (20).

9. An apparatus according to claims 1-8, **characterized in that** the pressure on the liquid is provided by an external impact on the reservoir (7).

10. An apparatus according to claim 9, **characterized in that** the external pressure impact on the reservoir (7) is provided by means of an external impact from a pressure bag (5) which is arranged in a chamber (6) together with the reservoir (7).

11. An apparatus according to claim 10, **characterized in that** the pressure bag (5) is supplied with pressure from an air pump (1), and that a safety valve (3) is arranged between the pressure bag (5) and the air pump.

12. An apparatus according to claim 10, **characterized in that** an electrically controlled pressure relief valve (4) is additionally arranged between the air pump (1) and the pressure bag (5).

13. An apparatus according to claims 10-12, **characterized in that** a pressure control in the form of a pressostat (2) is arranged in the connection between the pressure bag (5) and the safety valve (3).

14. An apparatus according to claims 1-13, **characterized in that** a shield (14) is arranged around the discharge branch (13).

## Patentansprüche

1. Gerät zur Spülung von peripheren Organen in Menschen und Tieren, wobei Flüssigkeit von einem Speicher (7) unter Druck durch eine Leitung (10) od. dgl. zu einer Abflussverzweigung (13) zu den peripheren Organen gefördert wird, wobei ein zur Pulsierung der von der Abflussverzweigung (13) abfliessenden Flüssigkeit vorgesehener Pulsator (9) zwischen dem Speicher (7) und der Abflussverzweigung (13) angeordnet ist, **dadurch gekennzeichnet, dass** das Gerät zur Steuerung des Druckes einen Druckschalter aufweist, und dass der Pulsator und der Druckschalter für die gegenseitig unabhängige Einstellung des Druckes und der Pulsation individuell regelbar sind.

2. Gerät nach Anspruch 1, **dadurch gekennzeichnet, dass** die Auslassdüse eine handgehaltene Einheit (11) aufweist, durch die die Menge und die Strahlenform der Flüssigkeit gesteuert werden kann, wobei die handgehaltene Einheit (11) eine Rollenverschlussanordnung (12) aufweist, und die Abflussdüse (13) ein oder mehrere Löcher aufweist.

3. Gerät nach den Ansprüchen 1-2, **dadurch gekennzeichnet, dass** der Speicher (7) aus einem flüssigkeitsdichten, vorzugsweise aus einem Kunststoff wie PVC oder Polyurethan hergestellten Sack dargestellt wird.

4. Gerät nach den Ansprüchen 1-3, **dadurch gekennzeichnet, dass** die handgehaltene Einheit (11) in einem verstellbaren, vorzugsweise aus einem transparenten Material wie PVC oder Polyurethan hergestellten Schirm endet.

5. Gerät nach den Ansprüchen 1-4, **dadurch gekennzeichnet, dass** die den Speicher (7) mit der handgehaltenen Einheit (11) verbindende Leitung (10) aus einem elastischen und vorzugsweise transparenten Material wie Silicongummi, Polyurethan oder PVC hergestellt ist.

6. Gerät nach Anspruch 1-5, **dadurch gekennzeichnet, dass** der Pulsator (9) für einen Einfluss auf die Leitung (10) durch eine pulsierende ganze oder teilweise Zusammenklemmen davon vorgesehen ist.

7. Gerät nach den Ansprüchen 1-6, **dadurch gekennzeichnet, dass** es mit einer für die Heizung der Flüssigkeit im Flüssigkeitsspeicher auf eine vorbestimmte Temperatur unter Beibehaltung der Flüssigkeitstemperatur innerhalb eines gegebenen Toleranzgebietes fähigen Errichtung versehen ist, und dass eine Temperatur zwischen 20 und 40°C, vorzugsweise 37°C, aufrechterhalten wird.

8. Gerät nach Anspruch 6 oder 7, **dadurch gekennzeichnet, dass** das Verklemmen der Leitung (10) durch einen Verschlussmechanismus (16, 17, 18) durchgeführt wird, der durch den Einfluss eines Elektromagneten (18) oder durch einen elektrisch oder hydraulisch gesteuerten Motor oder durch eine vorzugsweise steuerbare Rollenpumpe (20, 21, 22) mit einer oder mehreren Rollen (20) versetzt wird.

9. Gerät nach den Ansprüchen 1-8, **dadurch gekennzeichnet, dass** der Druck auf die Flüssigkeit durch einen äusseren Einfluss auf den Speicher (7) ausgelöst wird.

10. Gerät nach Anspruch 9, **dadurch gekennzeichnet, dass** der äussere Druckeinfluss auf den Speicher (7) durch einen ausseren Einfluss von dem zusammen mit dem Speicher in einer Kammer (6) vorgesehenen Drucksack (5) ausgelöst wird.

11. Gerät nach Anspruch 10, **dadurch gekennzeichnet, dass** der Drucksack (5) mit Druck von einer Luftpumpe (1) versorgt wird, und dass ein Sicherheitsventil (3) zwischen dem Drucksack (5) und der Luftpumpe angeordnet ist.

12. Gerät nach Anspruch 10, **dadurch gekennzeichnet, dass** zusätzlich ein elektrisch gesteuertes Überdruckventil (4) zwischen der Luftpumpe (1) und dem Drucksack (5) angeordnet ist.

13. Gerät nach Anspruch 10-12, **dadurch gekennzeichnet, dass** eine Drucksteuerung in der Form eines Druckschalters (2) in dem Übergangsstück zwischen dem Drucksack (5) und dem Sicherheitsventil (3) angeordnet ist.

14. Gerät nach Anspruch 1-13, **dadurch gekennzeichnet, dass** ein Schirm um die Abflussverzweigung herum angeordnet ist.

## Revendications

1. Appareil destiné à rincer des organes périphériques chez l'homme ou chez l'animal, dans lequel un liquide est transporté d'un réservoir (7) sous pression à une section de décharge (13) à travers un tube (10) ou de ce genre aux organes périphériques, dans lequel un pulsateur (9) est aménagé entre le réservoir (7) et la section de décharge (13), ledit pulsateur est adapté pour pulser le liquide déchargé de la section de décharge (13), **caractérisé en ce que** l'appareil comprend un pressostat pour contrôler la pression et que le pulsateur et le pressostat sont individuellement réglables pour une régulation mutuellement indépendant de la pression et la pulsation.

2. Appareil selon la revendication 1, **caractérisé en ce que** la buse de décharge comprend une unité portative (11) à partir de laquelle le volume et la forme du jet de liquide peuvent être réglés, ladite unité portative (11) ayant un dispositif de fermeture à rouleau (12), et que la buse de décharge (13) comporte une ou plusieurs ouvertures.

3. Appareil selon les revendications 1 à 2, **caractérisé en ce que** le réservoir (7) prend la forme d'un sac étanche préférablement en matière plastique, par exemple en PVC ou en polyuréthane.

4. Appareil selon les revendications 1 à 3, **caractérisé en ce que** l'unité portative (11) est terminée par un bouclier réglable (14) réalisé de préférence d'un matériau transparent, par exemple le PVC ou le polyuréthane.

5. Appareil selon les revendications 1 à 4, **caractérisé en ce que** le tube (10) reliant le réservoir (7) à l'unité portative (11) est réalisé d'un matériau flexible et de préférence transparent, par exemple le caoutchouc de silicone, le polyuréthane ou le PVC.

6. Un appareil selon les revendications 1 à 5, **caractérisé en ce que** le pulsateur (9) est adapté pour exercer une pression pulsatoire complète ou partiale sur le tube (10).

7. Appareil selon les revendications 1 à 6, **caractérisé en ce qu'**il est réalisé avec un assemblage capable de faire chauffer le liquide dans le réservoir de liquide jusqu'à une température prédéterminée et de garder la température du liquide dans une intervalle de tolérance donnée, et que la température maintenue est de préférence de 20 à 40 degrés Celsius, et plus de préférence de 37 degrés Celsius.

8. Appareil selon la revendication 6 ou 7, **caractérisé en ce que** la pression du tube (10) est réalisée par un méchanisme de fermeture (16, 17, 18) activé par l'action d'un électro-aimant (18) ou par un moteur contrôlé de manière éléctrique ou hydraulique ou pneumatique ou par l'usage d'une pompe à rouleaux de préférence réglable (20, 21, 22) ayant un ou plusieurs rouleaux (20).

9. Appareil selon les revendications 1 à 8, **caractérisé en ce que** la pression sur le liquide est réalisée par une action extérieure sur le réservoir (7).

10. Appareil selon la revendication 9, **caractérisé en ce que** l'action de pression extérieure sur le réservoir (7) est réalisée par une action extérieure d'un sac de pression (5) arrangé dans une chambre (6) avec le réservoir (7).

11. Appareil selon la revendication 10, **caractérisé en ce que** le sac de pression (5) est alimenté d'une pression venant d'une pompe à air (1), et qu'une soupape de sécurité (3) est aménagée entre le sac de pression (5) et la pompe à air.

12. Appareil selon la revendication 10, **caractérisé en ce qu'**une soupape de surpression (4) contrôlée de manière électrique est encore aménagée entre la pompe à air (1) et le sac de pression (5).

13. Appareil selon les revendications 10 à 12, **caractérisé en ce qu'**un contrôle de pression réalisé en forme d'un pressostat (2) est aménagé entre le sac de pression (5) et la soupape de sécurité (3).

14. Appareil selon les revendications 1 à 13, **caractérisé en ce qu'**un bouclier (14) est aménagé autour de la section de décharge (13).
